# EUROPEAN PATENT SPECIFICATION

(11) **EP 3 157 486 B1**
(45) Date of publication and mention of the grant of the patent: **05.05.2021**
(21) Application number: 15810320.0
(22) Date of filing: 12.06.2015
(51) Int. Cl.: A61F 13/49, A61F 13/496, A61F 13/534

(54) **STRETCH BREATHABLE PROTECTIVE ABSORBENT ARTICLE USING BILAMINATE**
ATMUNGSAKTIVER DEHNBARER SAUGFÄHIGER SCHUTZARTIKEL MIT BILAMINAT
ARTICLE ABSORBANT PROTECTEUR RESPIRANT ÉTIRABLE UTILISANT UN STRATIFIÉ À DEUX COUCHES

(30) Priority: 17.06.2014 US 201414306325
(43) Date of publication of application: 26.04.2017
(73) Proprietor: Medline Industries, Inc., Mundelein, IL 60060 (US)
(72) Inventor: NELSON, Christopher, Plymouth, WI 53073 (US)
(74) Representative: Gaunt, Thomas Derrick
(86) International application number: PCT/US2015/035467
(87) International publication number: WO 2015/195468

(56) References cited:
- WO-A1-2015/164170
- WO-A1-2015/164173
- US-A1- 2004 102 755
- US-A1- 2004 133 180
- US-A1- 2005 027 279
- US-A1- 2007 239 131
- US-A1- 2008 287 899
- US-A1- 2010 076 390
- US-A1- 2010 163 161
- US-A1- 2010 168 705
- US-A1- 2014 130 956
- US-B2- 6 994 761

## Description

### FIELD OF THE INVENTION

The present invention relates a disposable absorbent article in an underwear or pull-up style. More particularly, the invention relates to protective underwear that makes use of breathable laminate material having two layers.

### BACKGROUND OF THE INVENTION

Millions of people of all ages suffer from incontinence of the bowel or bladder. Whether an infant, adult, or elderly person, the underlying cause of incontinence varies but the method of treatment typically involves use of absorbent article products. Adult incontinent briefs, disposable diapers and underpads can alleviate some of the emotional and physical discomfort of incontinence by absorbing and containing liquid and other discharges from the human body to prevent body and clothing soiling.

A disadvantage of known disposable undergarments is that they are often constructed from materials that are designed to capture urine and other exudates and prevent leakage, but are not breathable. Consequently, moisture may become trapped between the wearer and the disposable undergarment leading to discomfort and irritation. Further, as disposable undergarments are intended to replace traditional undergarments, disposable undergarments must be constructed to permit the wearer to be repeatedly put-on and pull-off the garment as necessary until such time as the garment is ready for disposal.

Disposable protective underwear products are known in the art. Such disposable underwear products rely on retractive forces that are provided by elastics, such as spandex strands. It is also known to use stretch elastic laminates that replace the spandex strands so as to provide better a fit to the wearer and improved discretion. Some products are created from a coextruded elastic layer made during the nonwoven manufacturing process to provide a product with improved breathability.

Widlund, et al., US Patent No. 6,375,646 teaches a disposable diaper including an elongated absorbent pad, inner and outer casing layers and an elastically stretchable region in at least one of the front and back portions of the disposable diaper. The crotch portion of the disposable diaper is not stretchable. The combined stretchable and non-stretchable regions are designed to hold the absorbent material against the wearer's body to prevent leakage.

Norrby, et al., U.S. Patent No. 8,298,205 teaches an elastically stretchable laminate that includes a first non-elastic nonwoven web, a second non-elastic nonwoven web and an elastic film between the first and the second nonwoven webs. The laminate is rendered elastic in a first direction by incremental stretching and partial tearing of the first and second nonwoven webs.

Thorson, et al., U.S. Patent Application Publication No. 2011/0098668, teaches a disposable absorbent garment employing elastomeric film laminate body panels. The laminate can include an elastomeric film and nonwoven layers, and inner and outer surfaces adhered to nonwoven and elastomeric film layers.

Stablefeldt, et al., U.S. Patent Application Publication No. 2010/0168705, teaches disposable absorbent garments employing elastomeric film laminates with deactivated regions. A portion of the disposable garment includes laminated elastomeric and non-elastomeric polymeric film layers and a nonwoven layer. An absorbent member extends partially through the laminated layers.

Gilgenback U.S. Patent Application Publication No. 2010/0163161 teaches a process for making disposable absorbent garments employing elastomeric film laminates with deactivated regions. A portion of the disposable garment includes laminated elastomeric and non-elastomeric polymeric film layers and a nonwoven layer. An absorbent member extends partially through the laminated layers.

Kielpikowski, et al., U.S. Patent No. 4,842,596, teaches a method for making a breathable elastic fabric composite and personal article incorporating same. A liquid impermeable elastomeric film is sandwiched between pairs of nonwoven sheets. The elastomeric film is a partially stretched condition and bonded to the nonwoven sheets. The resulting laminated sheets create gathers that form breathable apertures.

Klemp, et al., U.S. Patent No. 6,994,761 teaches a disposable diaper and process for making the same. The diaper includes inner and outer portions that are ultrasonically bonded to create the vent sites or apertures through a layer of stretchable, breathable material.

Morrell-Schwartz, et al U.S. Patent Application Publication No. 2008/0287899 teaches a disposable diaper including an ultrasonically bonded stretchable film.

Nelson International Patent Application Publication No. WO2015/164170 teaches a disposable diaper including a bi-laminate that is bonded in a first zone and is glued in a second zone.

Nelson International Patent Application Publication No. WO2015/164173 teaches a disposable diaper including a tri-laminate that is bonded in a first region and is glued in a second region

### SUMMARY OF THE INVENTION

The invention is set out in the appended set of claims. Portions of the present disclosure are not within the scope of the claimed invention.

### BRIEF DESCRIPTION OF THE FIGURES

Advantages of the invention will become apparent upon reading the following detailed description and upon reference to the drawings.
FIG. 1 is a top plan view of laminate portions of an absorbent article in a substantially flat un-contracted position according to an embodiment of the present disclosure.
FIG. 2 is a cross-sectional view of the laminate of FIG. 1 along sectional line A-A.
FIG. 3 is a cross-sectional view of a breathable laminate according to the present disclosure.
FIG. 4 is a top view of the laminate of FIG. 3.
FIG. 5 is a top plan view of an embodiment of the absorbent article of FIG. 1 in a substantially flat un-contracted position and further including leg and waist elastics.
FIG. 6 is an alternative embodiment of the absorbent article of FIG. 5.
FIG. 7 is a top plan view of an embodiment of the absorbent article of FIG. 1 in a substantially flat un-contracted position and further including a covering nonwoven layer.
FIG. 8 is a cross-sectional view of the absorbent article of FIG. 7 along sectional line B-B.
FIG. 9 is a cross-sectional view of an embodiment of a laminate used in a portion of the absorbent article illustrated in FIG. 7.
FIG. 10 is a cross-sectional view of an embodiment of a laminate used in a further portion of the absorbent article illustrated in FIG. 7.
FIG. 11 is a top plan view of an embodiment of the absorbent article of FIG. 1 in a substantially flat un-contracted position and further including an absorbent assembly.
FIG. 12 is an exploded perspective view of an embodiment of the absorbent article of FIG. 11.
FIG. 13 is a cross-sectional view of a breathable laminate for use in a further in an absorbent article as disclosed herein.
FIG. 14 is a top view of the laminate of FIG. 13.

### DETAILED DESCRIPTION

Absorbent articles as described herein generally include a moisture-pervious inner layer, an absorbent layer, and a moisture-impervious outer layer. Although the remainder of the description will be specifically directed to adult incontinence articles, such as disposable diapers or briefs, it is to be understood that the embodiments may also be implemented using other absorbent articles and that the properties and uses described below apply to these other absorbent articles as well. Throughout this application, the terms absorbent article, diaper or brief are used interchangeably. However, it should be understood that the terms diaper or brief are intended to include other absorbent articles, such as training pants, incontinence pads, etc., as would be understood by one of ordinary skill in the art.

As used in the description herein and throughout the claims, the following terms take the meanings explicitly associated herein, unless the context clearly dictates otherwise: the meaning of "a," "an," and "the" includes plural reference, the meaning of "in" includes "in" and "on." Relational terms such as first and second, top and bottom, proximal and distal, and the like may be used solely to distinguish one entity or action from another entity or action without necessarily requiring or implying any actual such relationship or order between such entities or actions.

As used herein, the following terms have the following meanings:

"Attach" and its derivatives refer to the joining, adhering, connecting, bonding, sewing together, or the like, of two elements. Two elements will be considered to be attached together when they are integral with one another or attached directly to one another or indirectly to one another, such as when each is directly attached to intermediate elements. "Attach" and its derivatives include permanent, releasable, or refastenable attachment. In addition, the attachment can be completed either during the manufacturing process or by the end user.

"Bond" and its derivatives refer to the joining, adhering, connecting, attaching, sewing together, or the like, of two elements. Two elements will be considered to be bonded together when they are bonded directly to one another or indirectly to one another, such as when each is directly bonded to intermediate elements. "Bond" and its derivatives include permanent, releasable, or refastenable bonding.

"Connect" and its derivatives refer to the joining, adhering, bonding, attaching, sewing together, or the like, of two elements. Two elements will be considered to be connected together when they are connected directly to one another or indirectly to one another, such as when each is directly connected to intermediate elements. "Connect" and its derivatives include permanent, releasable, or refastenable connection. In addition, the connecting can be completed either during the manufacturing process or by the end user.

"Breathable" when used in describing a layer or multi-layer laminate means that the layer has the ability to allow moisture vapor to be transmitted through the material. Breathable layers may be air permeable, but it is not necessary to be air permeable to be breathable. In addition, breathable layers may be liquid permeable or liquid impermeable.

"Disposable" refers to articles that are designed to be discarded after a limited use rather than being laundered or otherwise restored for reuse.

The terms "disposed on," "disposed along," "disposed with," or "disposed toward" and variations thereof are intended to mean that one element can be integral with another element, or that one element can be a separate structure bonded to or placed with or placed near another element.

"Fiber" refers to a continuous or discontinuous member having a high ratio of length to diameter or width. Thus, a fiber may be a filament, a thread, a strand, a yarn, or any other member or combination of these members.

"Layer" when used in the singular can have the dual meaning of a single element or a plurality of elements.

"Liquid impermeable," when used in describing a layer or multi-layer laminate means that liquid, such as urine, will not pass through the layer or laminate, under ordinary use conditions, in a direction generally perpendicular to the plane of the layer or laminate at the point of liquid contact.

"Liquid permeable" refers to any material that is not liquid impermeable.

"Member" when used in the singular can have the dual meaning of a single element or a plurality of elements.

"Nonwoven" and "nonwoven web" refer to materials and webs of material that are formed without the aid of a textile weaving or knitting process. For example, nonwoven materials, fabrics or webs have been formed from many processes such as meltblowing processes, spunbonding processes, air laying processes, and bonded carded web processes.

These terms may be defined with additional language elsewhere in the specification.

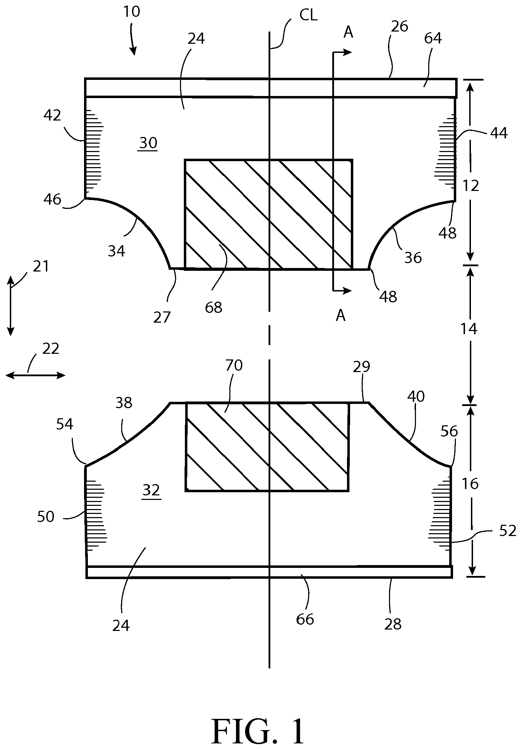

FIGS. 1, 5-7 and 9 illustrate a plan view of the absorbent article 10 in a substantially flat un-contracted state. As shown in these figures, the absorbent article 10 generally consists of several layers, including an inner layer, an absorbent layer, and an outer layer. The inner layer faces a wearer and contacts the skin of the wearer when the absorbent article 10 is secured to the wearer. The inner layer may comprise a topsheet that is composed of a moisture-pervious fabric suitable to allow bodily discharge to pass through the inner layer and be absorbed by the absorbent layer. Non-limiting examples of materials suitable to form the topsheet include polypropylene, polyethylene, polyester, materials having hydrophobic properties, combinations thereof and/or the like. Additionally, the topsheet can be treated with a hydrophilic finish to improve pass through of liquids to diaper layers beneath the inner layer. Non-limiting examples of suitable hydrophilic finishes include stearic acid, melamine-based chemicals, fluorocarbon chemicals, and silicon based chemicals.

The plan view of FIGS. 1, 5-7 and 9 is shown from the top or patient contacting side of the absorbent article. As illustrated in these figures, a particular embodiment of a disposable absorbent article 10 defines a longitudinal direction 21 parallel to a centerline CL and a transverse direction 22 perpendicular to the longitudinal direction. The absorbent article comprises a front section 12, a rear section 16, and a crotch section 14.

Referring to FIG. 1, the absorbent article includes a film layer 24 comprising a laminate film. The laminate film may be divided into two sections such that the film layer 24 forms at least part of the front section 12 and rear section 16. The front film section 30 is spaced apart from the rear film section 32 such that they are separated in the crotch section 14.

The front film section 30 defines a front end edge 26 and a front crotch edge 27 parallel to and longitudinally spaced from the front end edge 26. The rear film section 32 defines a rear end edge 28 longitudinally opposite the front end edge 26 and a rear crotch edge 29 parallel to and longitudinally spaced from the rear end edge 28. The front film section defines opposed front leg edges 34 and 36, and the rear film section defines opposed rear leg edges 36 and 38.

The front film section 30 further defines first and second transversely opposed front side edges 42 and 44. The first front side edge 42 extends in the longitudinal direction 21 from the front end edge 26 to a front intersection point 46 where the first front side edge intersects the first front leg edge 34. The second front side edge 44 extends in the longitudinal direction 21 from the front end edge 26 to a front intersection point 48 where the first front side edge intersects the second front leg edge 36. The rear film section 32 also defines first and second transversely opposed back side edges 50 and 52. The first back side edge 50 extends in the longitudinal direction 21 from the back end edge 28 to a rear intersection point 54 where the first rear side edge 50 intersects the first rear leg edge 38, and the second back side edge 52 extends in the longitudinal direction 21 from the back end edge 28 to a rear intersection point 56 where the second rear side edge 52 intersects the second rear leg edge 40.

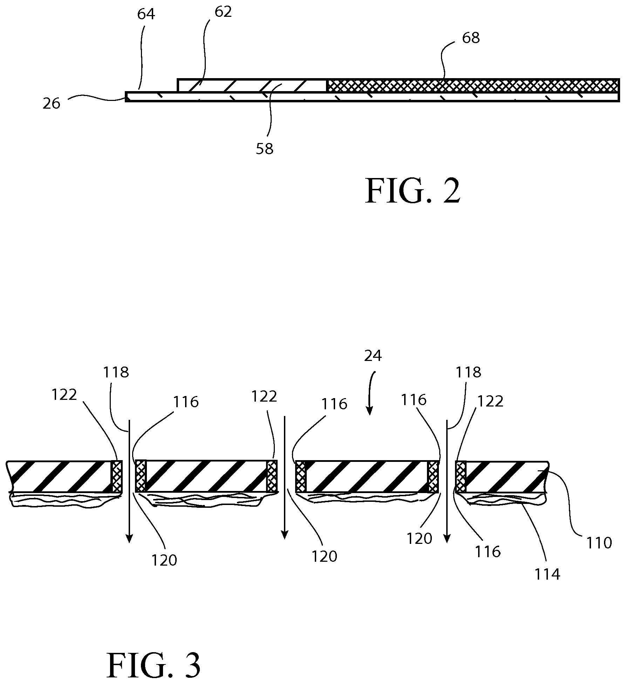

In particular embodiments, the front section 30 is constructed at least in part of a laminate 24 that comprises a polymeric film layer 62 and at least one nonwoven layer 58 (FIG. 2), wherein both the polymeric film layer 62 and the nonwoven layer 58 extend substantially throughout the area of the laminate 24.

In its completed form as used by a wearer, the absorbent article includes a first side seam at which the first front side edge 42 is attached to the first back side edge 50 and which defines a first side seam length. The article further includes a second side seam at which the second front side edge 44 is attached to the second back side edge 52 and which defines a second side seam length. The article is accordingly formed into a brief or pull-up style disposable absorbent article.

FIG. 2 shows a cross-sectional view of the laminate 24 along line A-A. In one embodiment, the laminate 24 comprises a first nonwoven layer 58 superposed on the bottom surface of the polymeric film 62 such that the polymeric film 62 and the first nonwoven layer 58 extend substantially through the area of the laminate 24. The polymeric film layer may be a block copolymer. A portion 64 of the bottom or outer layer of nonwoven 58 may extend beyond the polymeric film 62 along front end edge 26. A further portion 64 of the bottom or outer layer of nonwoven 58 may extend beyond the polymeric film 62 along front end edge 26.

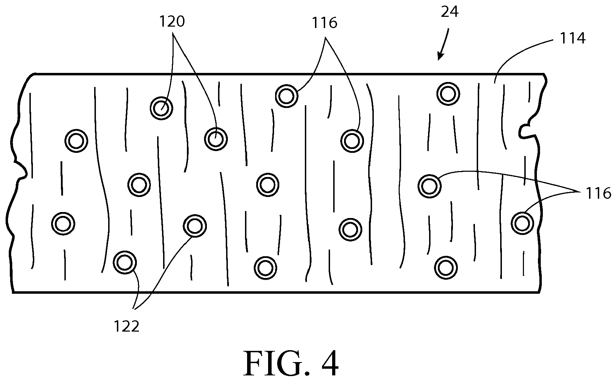

As shown in FIGS. 3-4, the laminate 24 may be formed of a breathable cloth-like elastic nonwoven laminar fabric by attaching a liquid impermeable and non-self-adhering elastomeric film or nonwoven carrier sheet 110 to a nonwoven facing sheet 114 and bonding the facing sheet 114 and elastomeric film 110 together by autogenous bonds, such as ultrasonically or thermally-generated bonds at spaced apart sites 116, thereby forming breathable apertures 120 through the carrier sheet which laminate the carrier and facing sheet together at the spaced apart sites 116.

The facing sheet 114 and the elastomeric film 110 are ultrasonically bonded at sites 116. The ultrasonic bonding process creates a bond region 122 where the material from the facing sheet 114 and elastomeric film 110 mix together to form a bond. The ultrasonic bonding process may be configured such that it generates a through passage 120 generally within the confines of the bond region 122 in order to provide for the passage of water vapor 118 and give breathability to the laminate 24.

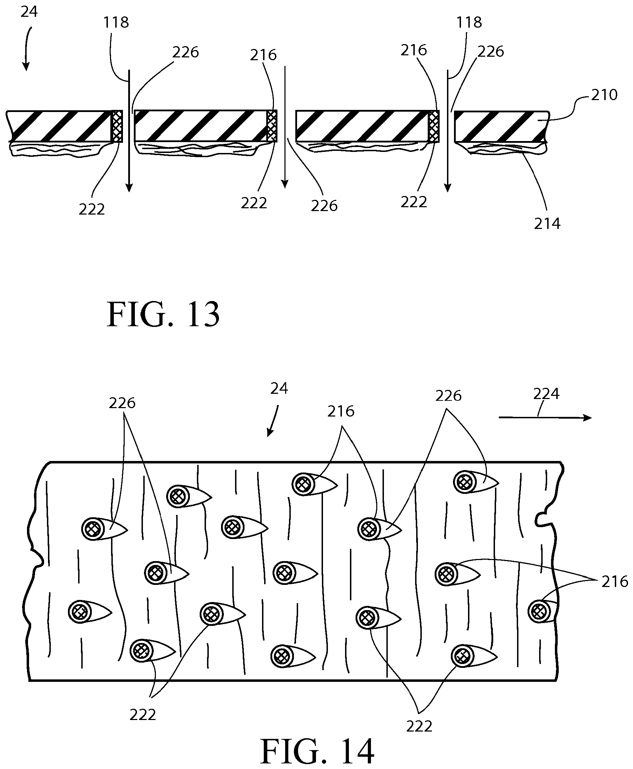

In an alternative embodiment as illustrated in FIGS. 13-14, the facing sheet 214 and the elastomeric film 210 are ultrasonically bonded at sites 216. The ultrasonic bonding process creates a bond region 222 where the material from the facing sheet 214 and elastomeric film 210 mix together to form a bond. The ultrasonic bonding process may be conducted while the sheet is moving in a direction 224 such that a trailing tear 226 forms in the laminate generally outside and adjacent to the bond region 222. These trailing tears 226 provide through openings for the passage of water vapor 118 and give breathability to the laminate 24. While the bond regions 222 and trailing tears 226 are shown in FIGS. 13-14 as being of a size and shape sufficiently large to illustrate the structure of the bonded laminate, one of skill in the art will understand that these may be of any appropriate size and shape and may be sufficiently small that they are not be readily apparent without the use of magnification.

Alternatively, the laminate 24 can also be constructed such that the web is not made breathable during the ultrasonic laminating process, but rather has breathability imparted through a needling, slitting or die treatment process after formation of the complete laminate.

Referring again to FIG. 1, at least a portion 68 of the front film section 30 is non-elastomeric, and at least a portion 70 of the rear film section 32 is non-elastomeric. In FIGS. 1, 5-7 and 9 non-elastomeric or partially elastomeric regions are indicated by a pattern of hash lines, which lines are continuous if the non-elastomeric regions are exposed, and which lines are dashed if the non-elastomeric regions are concealed by an overlying component. In one preferred approach, as shall be described in more detail below, the entire laminate 24 is constructed of an elastomeric film laminate which includes an elastomeric film layer and at least one nonwoven facing layer, and a portion of the laminate has been "deactivated" or "deadened" to render it non-elastomeric.

As used herein, "elastomeric" refers to a material or composite which can be elongated by at least 50 percent of its relaxed length and which will recover, upon release of the applied force, at least 50 percent of its elongation. It is generally preferred that the elastomeric material or composite be capable of being elongated by at least 100 percent, more preferably by at least 200 percent, of its relaxed length and recover, upon release of an applied force, at least 50 percent of its elongation. "Non-elastomeric" refers to a material or composite that is non-extensible, or that is extensible but will recover no more than 20 percent of its elongated length after release of an applied elongating force. "Non-extensible" refers to a material that cannot stretch or extend by more than 25 percent of its relaxed length without fracture upon application of a biasing force. "Partially elastomeric" refers to a material or composite which can be elongated by at least 50 percent of its relaxed length and which will recover, upon release of the applied force, more than 20 percent but less than 50 percent of its elongation.

"Deactivated" as used herein to describe a material, region of a material, or regions of a material means that the material, region, or regions of material has been treated in some way to substantially destroy the elastic properties of the material, region, or regions, rendering the material, region, or regions non-elastomeric.

Deactivation of the non-elastic portions 68, 70 may be accomplished by a deactivation unit to create deactivated regions in the elastomeric film laminate 24. The deactivation can be accomplished by any of a variety of means. Frequently, some form of energy is applied to deactivate the non-elastic regions 68, 70, such as pressure, heat, ultrasonic energy, combinations thereof, and the like. Techniques employing pressure, heat, and ultrasonic energy are known in the art. The deactivation can occur in a variety of patterns. For example, the deactivating energy could be applied in a solid pattern, a series of vertical stripes, horizontal stripes, or diagonal stripes, a series of squares or dots, or other suitable pattern.

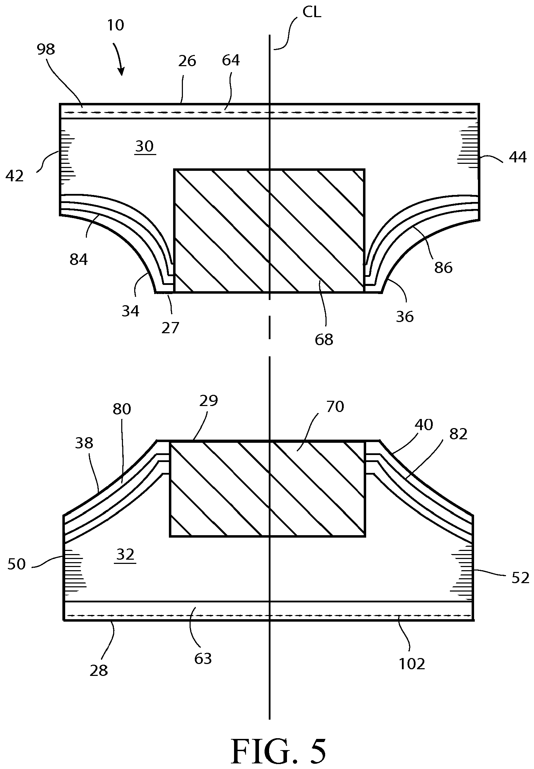

In embodiments of the present invention as illustrated in FIG. 5, the absorbent article 10 comprises a first rear leg elastic member 80 attached to an inside surface of the rear film section 32 adjacent at least a portion of the first rear leg edge 38, and a second back leg elastic member 82 to an inside surface of the rear film section 32 adjacent at least a portion of the second rear leg edge 40. In further embodiments, the absorbent article 10 comprises a first front leg elastic member 84 attached to an inside surface of the front film section 30 adjacent at least a portion of the first front leg edge 34, and a second front leg elastic member 86 attached to attached to an inside surface of the front film section 30 adjacent at least a portion of the second front leg edge 36. Each leg elastic member 80, 82, 84, 86 can comprise a single strand, ribbon, or strip of elastomeric material, or each can comprise two or more strands, ribbons, or strips, such as, for example, three strands (as depicted in FIG. 5). The leg elastic members 80, 82, 84, 86 may be glued in place or otherwise adhered to a top surface of nonwoven layer 58.

As illustrated in FIG. 5, rear leg elastic member 80, 82 may extend from side edges 50, 52 of the rear film section 32 along rear leg edges 38, 40 to side edges of the non-elastic portion 70 of the rear film section. Alternatively, the rear leg elastic member 80, 82 may extend across part or the entire non-elastic portion 70. Likewise, front leg elastic member 84, 86 may extend from side edges 42, 44 of the front film section 30 along front leg edges 34, 36 to side edges of the non-elastic portion 68 of the front film section. Alternatively, the front leg elastic member 84, 86 may extend across part or the entire non-elastic portion 68.

For example, the first rear leg elastic member 80 and the second rear leg elastic member 82 may form part of a single, integral back elastic member that extends from the first rear side edge 50 transversely over the non-elastic portion 70 to the second rear side edge 52. Similarly, in certain embodiments, the first front leg elastic member 84 and the second front leg elastic member 86 form part of a single, integral front elastic member that extends from the first front side edge 42 transversely over the non-elastic portion 68 to the second front side edge 44.

In embodiments, as illustrated in FIG. 5, the extension 64 of the outer nonwoven layer 58 (see FIGS. 1-2) of the front portion 30 may be folded over the top of inner nonwoven layer 60 to define the front end edge 26. A similar extension 63 of the outer layer of nonwoven of the rear laminate 32 may be folded over the top of inner nonwoven layer to define the rear end edge 28.

The absorbent article 10 may further include a front waist elastic member 98 positioned within the front fold 64 and a back waist elastic member 102 positioned within the back fold 63. In alternative embodiments, no front waist fold or back waist fold is included; in such embodiments, opposite end edges of the laminate sections 30, 32 would define the front end edge 26 and back end edge 28, respectively. Each waist elastic member 98, 102 may comprise a single strand, ribbon, or strip of elastomeric material, or each can comprise two or more strands, ribbons, or strips.

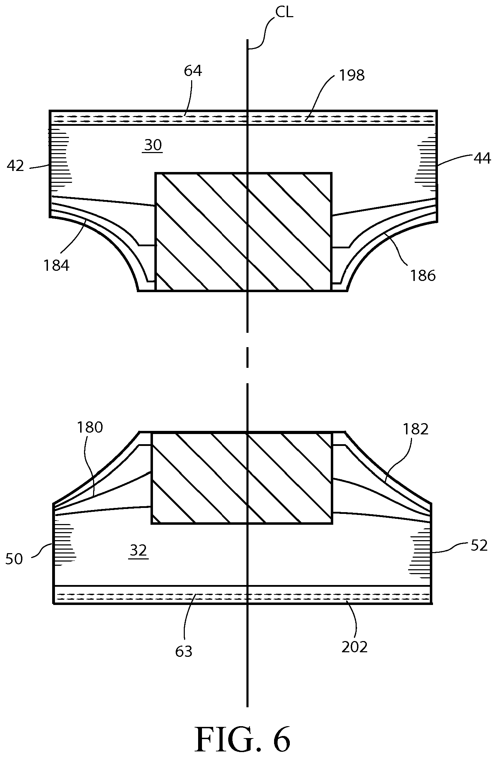

FIG. 6 illustrates further embodiments in which the leg elastic members 180, 182, 184, 186 comprises a series of elastic strands. The illustrated embodiment shows three such strands, but more or fewer strands may be used. The leg elastics may be applied in a curved fashion. At the side edges 42, 44, 50, 52 of the diaper, the leg elastics are generally parallel, and each of the independent leg elastics are then curved towards the respective non-elastic portions 68, 70 of the film sections 30, 32, and increasingly separated in distance from one another the closer the leg elastics get to the non-elastic film portions. Also as shown in FIG. 6, the waist elastic members 198, 202 may comprise multiple elastic strands.

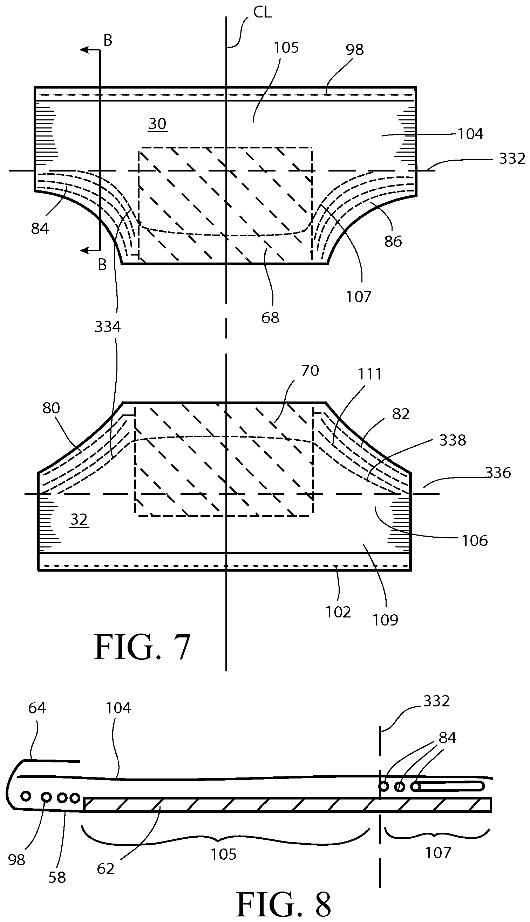

As illustrated in FIGS. 7-8, an additional covering nonwoven layer 104 may be attached to a top surface of the polymeric film layer 62 that comprises the top layer of the front laminate section 30. Additionally, a further covering nonwoven layer 106 may be attached to a top surface of the polymeric film layer 62 that comprises the top layer of the rear laminate section 32. The covering nonwoven layers 104, 106 are placed on top of laminate sections so that they at least in part cover the leg elastic elements. The covering nonwoven layers may be bonded to the film laminate 24 in the region 105 where the leg elastics 80, 82, 84, 86 are not present and may be glued to the elastics and/or the polymeric film layer 62 in region 107 where the elastics are located.

The front 30 and rear 32 laminate sections may each be separated into two or more areas that are characterized by the manner in which the covering nonwoven layers 104, 106 are attached to the polymeric film layer 62. The front section 30 may have a first bonded portion 105 in which the covering nonwoven 104 is attached to the film laminate 24 by autogenous bonds, such as ultrasonically or thermally-generated bonds at spaced apart sites. The rear section 32 may have a corresponding bonded portion 109.

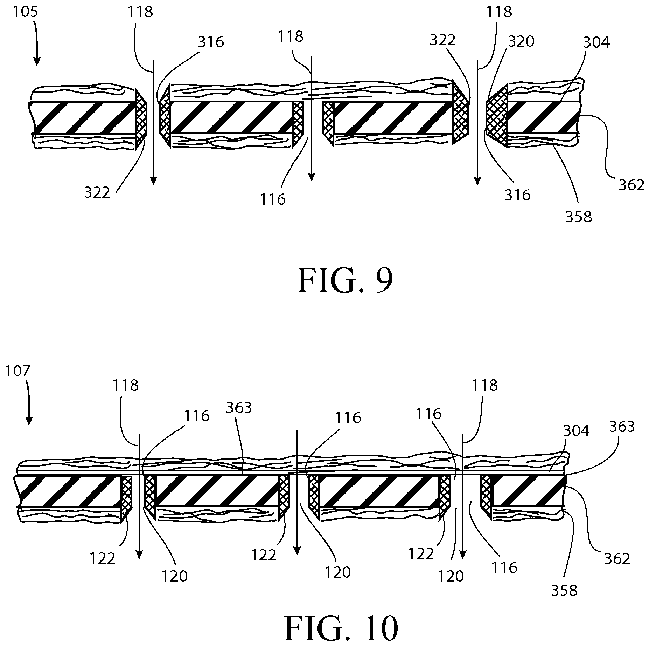

As illustrated in FIG. 9, the spaced apart bonding sites 316 of areas 105 form breathable apertures 320 through the outer nonwoven layer 358, polymeric film layer 362 and covering nonwoven layer 304. The ultrasonic bonding process creates a bond region 322 where the material from the outer facing sheet 358, polymeric film 362 and inner facing sheet 304 mix together to form a bond. The ultrasonic bonding process may be configured such that it generates a through passage 320 generally within the confines of the bond region 322 in order to provide for the passage of water vapor 118 and give breathability to the laminate 24. These bonding sites 316 may correspond to the boding sites 116 already present in the laminate 24 (which consists of outer facing nonwoven sheet 358 and polymeric film 362). Alternatively, the bonding sites 316 may be not align to the existing bond sites 116 such that the bonding sites 316 create new through passages 320 that pass through the three layers of the laminate. In some embodiments, this will result in previous bond sites 116 that are covered on the inside by the inner facing nonwoven layer 304. The inner facing layer 304 may be a breathable nonwoven material such that water vapor 118 may pass through previous bond sites 116 even though those bond sites are now covered by the inner facing layer 304.

Referring again to FIG. 7, the front section 30 may have a second adhered portion 107 in which the covering nonwoven 104 is attached to the film laminate 24 by an adhesive or lamination process. The rear section 32 may have a corresponding adhered portion 111.

As shown in FIG. 10, the covering nonwoven 304 may be attached to the polymeric layer 362 by application of an adhesive material 363. This adhesive may in a manner, such that it does not block some or all of the existing through passages 120. For example, the adhesive may be applied in a pattern such that it is applied to some of the through passages 120, but not others. Alternatively, the adhesive may be of a type or applied in such a manner that it does not fill or seal the existing through passages 120. Accordingly, provided that the inner facing layer 304 is a breathable material, water vapor 118 may pass through previous bond sites 116 even though those bond sites are now covered by the inner facing layer 304, and the breathability of these areas 107, 111 will be preserved.

The differently adhered areas 105, 107 of the front section 30 may be separated by a line 332 that runs transversely across the width of the absorbent article such that the upper waist area of the article fall generally within the first area 105 while the lower waist area and leg elastics 84, 86 fall generally within the second area 107. Alternatively, the differently adhered areas may be separated by a line 334 that more closely follows the contours of the leg elastics such that more of the waist area falls within the first area 105. Likewise, the differently adhered areas 109, 111 of the rear section 32 may be separated by a generally straight transverse line 336 or a more contoured line 338. It should be understood that the line separating these sections can be positioned and contoured as would be understood by one of skill in the art in order to performance, cost and other attributes of the absorbent article.

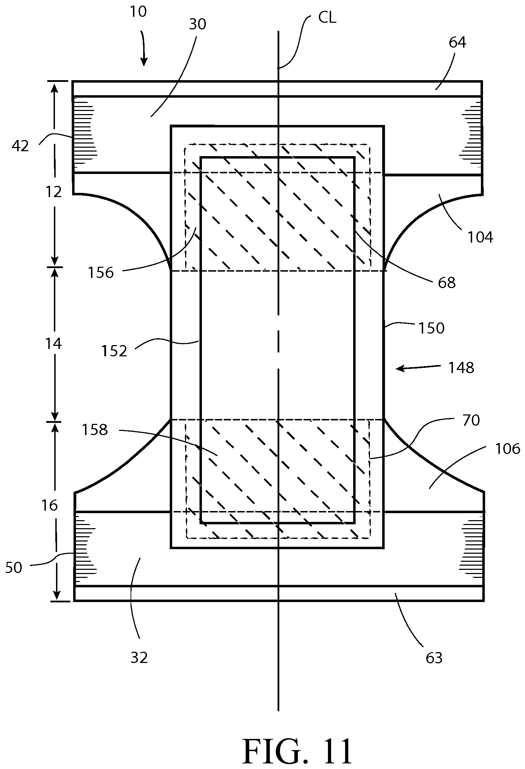

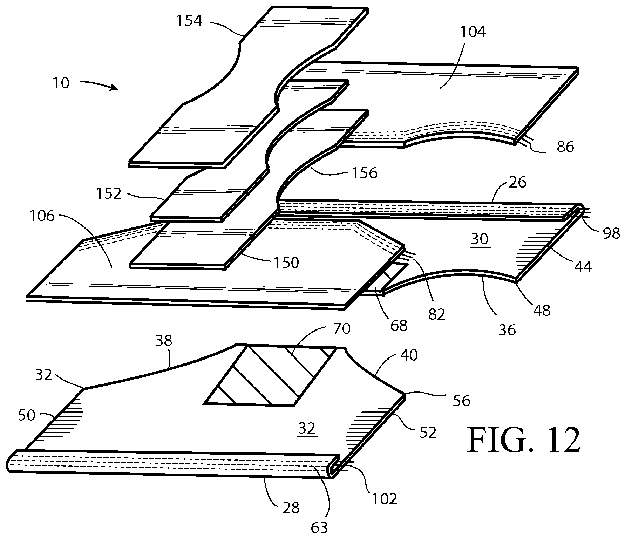

As illustrated in FIGS. 11-12, the absorbent article 10 also includes an absorbent assembly 148 that extends from the front section 12, across the crotch section 14, to the rear section 16. The absorbent assembly includes an absorbent core 152, and may include a topsheet 154 and a backsheet 150. (The topsheet 154 has been removed in FIG. 11 to more clearly show the position of the backsheet 150 and absorbent core 152.) The absorbent assembly 148 may be generally rectangular as shown in FIG. 11 or may comprise curved sections 166 to accommodate the wearer's legs as shown in FIG. 12. The absorbent core 152 may have an area that is smaller than the topsheet 154 and backsheet 150 such that the absorbent core is contained within the periphery of the absorbent assembly. The topsheet 154 and backsheet 150 may be bonded or otherwise adhered around a periphery of the absorbent assembly in order to capture the absorbent core 152 between the two sheets.

As shown in FIG. 11, the absorbent assembly 148 overlaps with the front section 30 to form a front overlapping zone 156, and the absorbent assembly 148 overlaps with the rear section 32 to form a rear overlapping zone 158. The periphery of the absorbent core 152 may be positioned completely within the front non-elastic portion 68 where the core overlaps with the front film section 30 and positioned completely within the rear non-elastic portion 70 where the core overlaps with the front film section 32. The backsheet 150 and topsheet (not shown) may also be positioned within the non-elastic portions 68, 70 in the overlapping zones, or may extend beyond the non-elastic portions as illustrated in FIG. 11.

In embodiments of the invention, the non-elastic portions 68, 70 may encompass more than 50% of the respective overlapping zones 156, 158. In other embodiments, more than 75%, and in further embodiments more than 90% of the area of the overlapping zones 156, 158 are non-elastomeric. In further embodiments, the entire of the overlapping zones are non-elastomeric. By adjusting the size of the non-elastic portions 68, 70 relative to the size of the absorbent core 152, the fit range of the article may be adjusted or the shape of the absorbent assembly may be defined in order to more readily capture exudate or prevent leaks.

In further embodiments, the non-elastic portions 68, 70 extend beyond the periphery of the absorbent core 152 or even beyond the backsheet 150. For example, non-elastic regions may be at least 10% larger, 20% larger, or in further embodiments 25% larger in area than the respective overlapped regions 156, 158. Providing non-elastic portions that are larger than their respective overlapping zones allows the process to accommodate any registration variability that may be present in the manufacturing process.

## Claims

1. An absorbent article, comprising:
a front laminate (30) section defining front end edge (26), a front crotch 5 edge (27) parallel to and longitudinally spaced from the front end edge (26), and first and second transversely opposed side edges (42, 44) extending in a longitudinal directions, the front laminate section (30) comprising a front non-elastic portion (68) adjacent to the front crotch edge (27),
a front elastic portion adjacent (24) to the front end edge (26), the front 10 laminate (30) sections further comprising:
a polymeric film (62, 362),
a first nonwoven layer (58, 358),
a rear laminate section (32) defining rear end edge (66), a rear crotch edge (29) parallel to and longitudinally spaced from the rear end edge (66), and 15 first and second transversely opposed side edges (50, 52) extending in a longitudinal directions, the rear laminate section (32) comprising a rear non-elastic portion (70) adjacent to the rear crotch edge (29) and a rear elastic portion adjacent (24) to the rear end edge (26); and
an absorbent assembly (148) extending longitudinally between the front 20 crotch edge (27) and the rear crotch edge (29), the absorbent assembly (148) comprising a topsheet (154), a back sheet (150) and an absorbent core (152) positioned between the topsheet (154) and backsheet (150);
**characterized in that** the front laminate section (30) further comprises a plurality of spaced apart ultrasonic bonding sites (116, 216) attaching the first nonwoven layer (58, 358) to a first side of the polymeric film (62, 362), wherein each bonding site creates a bond region 222 and forms an impermeable layer within the perimeter of the bonding site, and a plurality of breathable apertures (120, 320) through the polymeric film (62, 362), wherein the plurality of breathable apertures (120, 320) comprise tears (226) forming through passages in the front laminate that provide for the passage of water vapor (118) through the front laminate (30) with each tear (226) being formed in the laminate generally outside and adjacent to the bond region (222), and
a second nonwoven layer (104, 304) attached to a second side of the polymeric film (62, 362) opposite the first nonwoven layer (58, 358), wherein the second nonwoven layer (104, 304) is attached to the polymeric film (62, 362) by a first adherence mechanism in a first zone (105), wherein the first adherence mechanism is a plurality of spaced apart ultrasonic bonds (316), and a second adherence mechanism in a second zone (107), wherein the second adherence mechanism is an adhesive material (363).

2. The absorbent article of claim 1 wherein the absorbent assembly (148) overlaps (156, 158) with the front non-elastic portion (68) and the rear non-elastic portion (70) and does not overlap with the front elastic portion (24) and the rear elastic portion (24).

3. The absorbent article of claim 1 wherein the front laminate section (30) further defines a first leg edge (34), and the absorbent article further comprises:
a first leg elastic (84, 184) that is adhered to a surface of the front laminate section (30) and extends parallel and adjacent to the leg edge (34) from the side edge (42) to an edge of the front non-elastic portion (68); and
a second leg elastic that is adhered to a surface of the front laminate section (30) and that is increasingly separated in distance from the first leg elastic (84, 184) as the leg elastic extends from the side edge (42) to an edge of the front non-elastic portion (68).

4. The absorbent article of claim 3 wherein the leg elastic (84, 184) is positioned between the polymeric film (62, 362) and the second nonwoven layer (104).

5. The absorbent article of claim 1 wherein the polymeric film (62, 362) is an elastomeric film.

6. The absorbent article of claim 5 wherein the non-elastic portion (68) of the front laminate (30) is formed by deactivating a portion of the elastomeric film.

7. The absorbent article of claim 1 wherein leg elastics (84, 184) are positioned within the second zone (107).

8. An absorbent article, comprising:
a breathable front laminate section (30) defining front end edge (26), a front crotch edge (27) parallel to and longitudinally spaced from the front end edge (26), first and second transversely opposed side edges (42, 44) extending in a longitudinal directions, and first and second leg edges (34, 36), the front laminate section (30) comprising:
a polymeric film (62, 362),
a first nonwoven (58, 358) layer attached to a first side of the polymeric film (62, 362) at a plurality of spaced apart bonding sites (116, 216), wherein each bonding site creates a bond region 222 and forms an impermeable layer within the perimeter of the bonding site,
a second nonwoven layer (104) attached to a second side of the polymeric film (62, 362) opposite the first nonwoven layer (58, 358),
a front non-elastic portion (68) adjacent to the front crotch edge (27), and a front elastic portion (24) adjacent to the front end edge (26), and
a plurality of breathable apertures (120, 320) through the polymeric film (68);
a rear laminate section (32) defining rear end edge (28), a rear crotch edge (29) parallel to and longitudinally spaced from the rear end edge (28), first and second transversely opposed side edges (50, 52) extending in a longitudinal directions, and first and second leg edges (38, 40), the rear laminate section comprising (32) a rear non-elastic portion (70) adjacent to the rear crotch edge (29) and a rear elastic portion (24) adjacent to the rear end edge (28); and
an absorbent assembly (148) extending longitudinally between the front crotch edge (27) and the rear crotch edge (29), the absorbent assembly (148) comprising a topsheet (154), a back sheet (150) and an absorbent core (152) positioned between the topsheet (154) and backsheet (150);
**characterized in that** the second nonwoven (104) layer is attached to the polymeric film (62, 362) by a first adherence mechanism in a first zone (105), wherein the first adherence mechanism is a plurality of spaced apart ultrasonic bonds (316), and a second adherence mechanism in a second zone (107), wherein the second adherence mechanism is an adhesive material (363), and
the plurality of breathable apertures (120, 320) consists of a series of tears (226) forming through passages that provide for the passage of water vapor (118) through the front laminate (30) with each tear (226) being formed in the laminate generally outside and adjacent to the bond region (222).

9. The absorbent article of claim 8 wherein the bonding sites (116, 216) are ultrasonic bonding sites.

10. The absorbent article of claim 9 wherein the through passages are formed within a perimeter of the ultrasonic bonding site (116, 216).

11. The absorbent article of claim 9 wherein each tear (226) is formed outside of and adjacent to a perimeter of the ultrasonic bonding site (216).

12. The absorbent article of claim 8 wherein the front laminate section (30) further defines a first leg edge (34), and the absorbent article further comprises:
a first leg elastic (84, 184) that is adhered to a surface of the front laminate section (30) and extends parallel and adjacent to the leg edge (34) from the side edge (42) to an edge of the front non-elastic portion (68); and
a second leg elastic that is adhered to a surface of the front laminate section (30) and that is increasingly separated in distance from first leg elastic (84) as the leg elastics extend from the side edge (42) to an edge of the front non-elastic portion (68).

13. The absorbent article of claim 1 wherein the through passages are formed within a perimeter of the ultrasonic bonding site (116, 216).

## Patentansprüche

1. Ein absorbierender Artikel, umfassend:
Einen vorderen Schichtstoffabschnitt (30), der einen vorderen End-Rand (26), einen vorderen Schritt-Rand (27) parallel und längs, mit Zwischenraum versehen, zu dem vorderen End-Rand (26), und einen ersten und zweiten quer gegenüberliegenden Seiten-Rand (42, 44), die sich in Längsrichtung erstrecken, definiert, der vordere Schichtstoffabschnitt (30) umfasst einen vorderen nichtelastischen Abschnitt (68) am vorderen Schritt-Rand (27),
einen vorderen elastischen Abschnitt (24) am vorderen End-Rand (26), der vordere 1 oder Schichtstoffabschnitt (30) ferner umfassend:
eine Polymerfolie (62, 362),
eine erste Vliesschicht (58, 358),
einen hinteren Schichtstoffabschnitt (32), der einen hinteren End-Rand (66) definiert, einen hinteren Schritt-Rand (29) parallel und längs, mit Zwischenraum versehen, vom hinteren End-Rand (66), einen ersten und zweiten quer gegenüberliegenden Seiten-Rand (50, 52), die sich in Längsrichtung erstrecken, der hintere Schichtstoffabschnitt (32) umfasst einen hinteren nichtelastischen Abschnitt (70) am hinteren Schritt-Rand (29) und einen hinteren elastischen Abschnitt (24) am hinteren End-Rand (26); und
eine absorbierende Anordnung (148), die sich längs zwischen dem vorderen Schritt-Rand (27) und dem hinteren Schritt-Rand (29) erstreckt, die absorbierende Anordnung (148) umfasst eine Deckschicht (154), eine untere Lage (150) und eine absorbierende Kernschicht (152), die zwischen der Deckschicht (154) und der unteren Lage (150) positioniert ist;
**gekennzeichnet dadurch, dass** der vordere Schichtstoffabschnitt (30) ferner eine Vielzahl mit Zwischenräumen versehene Ultraschall-Bindungsstellen (116, 216) umfasst, die die erste Vliesschicht (58, 358) mit einer ersten Seite der Polymerfolie (62, 362) verbinden, wobei jede Bindungsstelle eine Bandregion (222) schafft und eine undurchlässige Schicht innerhalb des Umfangs der Bindungsstelle bildet, und eine Vielzahl von atmungsaktiven Öffnungen (120, 320) durch die Polymerfolie (62, 362), wobei die Vielzahl der atmungsaktiven Öffnungen (120, 320) Schlitze (226) umfassen, die Durchgangsöffnungen im vorderen Schichtstoff bilden, die den Wasserdampf (118) durch den vorderen Schichtstoff (30) lassen, wobei jeder Schlitz (226) im Allgemeinen außerhalb und anliegend an die Bandregion (222) geformt ist, und
eine zweite Vliesschicht (104,304), die an einer zweiten Seite der Polymerfolie (62,362) gegenüber der ersten Vliesschicht (58, 358) befestigt ist, wobei die zweite Vliesschicht (104, 304) an der Polymerfolie (62,362) durch einen ersten Haftmechanismus in einer ersten Zone (105) befestigt ist, wobei der erste Haftmechanismus eine Vielzahl von mit Abstand versehenen Ultraschall-Bindungsstellen (316) umfasst, und durch einen zweiten Haftmechanismus in einer zweiten Zone (107), wobei der zweite Haftmechanismus ein Haftmaterial (363) ist.

2. Der absorbierende Artikel nach Anspruch 1, wobei die absorbierende Anordnung (148) den vorderen nichtelastischen Abschnitt (68) und den hinteren nichtelastischen Abschnitt (70) überlappt (156, 158), aber den vorderen elastischen Abschnitt (24) und den hinteren elastischen Abschnitt (24) nicht überlappt.

3. Der absorbierende Artikel nach Anspruch 1, wobei der vordere Schichtstoffabschnitt (30) ferner einen ersten Bein-Rand (34) definiert, und der absorbierende Artikel ferner Folgendes umfasst:
Einen ersten Bein-Gummizug (84, 184), der an einer Oberfläche des vorderen Schichtstoffabschnitts (30) befestigt ist und parallel und anliegend am ersten Bein-Rand (34) vom Seiten-Rand (42) zu einem Rand des vorderen nichtelastischen Abschnitts (68) verläuft; und
einen zweiten Bein-Gummizug, der an einer Oberfläche des vorderen Schichtstoffabschnitts (30) befestigt ist und in zunehmendem Abstand vom ersten Bein-Gummizug (84, 84) getrennt ist, da der Bein-Gummizug vom Seiten-Rand (42) zu einem Rand des vorderen nichtelastischen Abschnitts (68) verläuft.

4. Der absorbierende Artikel nach Anspruch 3, wobei der Bein-Gummizug (84,184) zwischen der Polymerfolie (62, 362) und der zweiten Vliesschicht (104) positioniert ist.

5. Der absorbierende Artikel nach Anspruch 1, wobei die Polymerfolie (62, 362) eine Elastomerfolie ist.

6. Der absorbierende Artikel nach Anspruch 5, wobei der nichtelastische Abschnitt (68) des vorderen Schichtstoffs (30) durch die Deaktivierung eines Abschnitts der Elastomerfolie gebildet wird.

7. Der absorbierende Artikel nach Anspruch 1, wobei die Bein-Gummizüge (84, 184) innerhalb der zweiten Zone (107) positioniert sind.

8. Ein absorbierender Artikel, umfassend:
Einen atmungsaktiven vorderen Schichtstoffabschnitt (30), der einen vorderen End-Rand (26), einen vorderen Schritt-Rand (27) parallel und längs, mit Zwischenraum versehen, zu dem vorderen End-Rand (26), einen ersten und zweiten quer gegenüberliegenden Rand (42, 44), die sich in Längsrichtung erstrecken, und einen ersten und zweiten Bein-Rand (34, 36) definiert, der vordere Schichtstoffabschnitt (30) umfasst:
eine Polymerfolie (62, 362),
eine erste Vliesschicht (58, 358), die an einer ersten Seite der Polymerfolie (62, 362) an einer Vielzahl von mit Abstand versehenen Bindungsstellen (116, 216) befestigt ist, wobei jede Bindungsstelle eine Bandregion 222 und eine undurchlässige Schicht innerhalb des Umfangs der Bindungsstelle bildet,
eine zweite Vliesschicht (104), die an einer zweiten Seite der Polymerfolie (62, 362) gegenüber der ersten Vliesschicht (58, 358) befestigt ist,
einen vorderen nichtelastischen Abschnitt (68) am vorderen Schritt-Rand (27) und einen vorderen elastischen Abschnitt (24) am vorderen End-Rand (26), und
eine Vielzahl von atmungsaktiven Öffnungen (120, 320) durch die Polymerfolie (68);
einen hinteren Schichtstoffabschnitt (32), der einen hinteren End-Rand (28), einen hinteren Schritt-Rand (29) parallel und längs mit Zwischenraum versehen zum hinteren End-Rand (28) und einen ersten und zweiten quer gegenüberliegenden Seiten-Rand (50, 52), die sich in Längsrichtung erstrecken, definiert, der hintere Schichtstoffabschnitt (32) umfasst einen hinteren nichtelastischen Abschnitt (70) am hinteren Schritt-Rand (29) und einen hinteren elastischen Abschnitt (24) am hinteren End-Rand (28); und
eine absorbierende Anordnung (148), die sich längs zwischen dem vorderen Schritt-Rand (27) und dem hinteren Schritt-Rand (29) erstreckt, die absorbierende Anordnung (148) umfasst eine Deckschicht (154), eine untere Lage (150) und eine absorbierende Kernschicht (152), die zwischen der Deckschicht (154) und der unteren Lage (150) positioniert ist;
**gekennzeichnet dadurch, dass** die zweite Vliesschicht (104) an der Polymerfolie (62, 362) durch einen ersten Haftmechanismus in einer ersten Zone (105) befestigt ist, wobei der erste Haftmechanismus eine Vielzahl von mit Abstand versehenen Ultraschall-Bindungsstellen (316) umfasst, und einen zweiten Haftmechanismus in einer zweiten Zone (107),
wobei der zweite Haftmechanismus ein Haftmaterial (363) ist, und
die Vielzahl von atmungsaktiven Öffnungen (120, 320) besteht aus einer Reihe von Schlitzen (226), die Durchgangsöffnungen bilden, die für den Durchgang des Wasserdampfes (118) durch den vorderen Schichtstoff (30) sorgen, wobei jeder Schlitz (226) im Allgemeinen im Schichtstoff außerhalb und anliegend an die Bandregion (222) geformt ist.

9. Der absorbierende Artikel nach Anspruch 8, wobei die Bindungsstellen (116, 216) Ultraschall-Bindungsstellen sind.

10. Der absorbierende Artikel nach Anspruch 9, wobei die Durchgangsöffnungen innerhalb des Umfangs der Ultraschall-Bindungsstelle (116, 216) gebildet sind.

11. Der absorbierende Artikel nach Anspruch 9, wobei jeder Schlitz (226) außerhalb und am Umfang der Ultraschall-Bindungsstelle (216) gebildet ist.

12. Der absorbierende Artikel nach Anspruch 8, wobei der vordere Schichtstoffabschnitt (30) ferner einen ersten Bein-Rand (34) definiert, und der absorbierende Artikel ferner Folgendes umfasst:
einen ersten Bein-Gummizug (84, 184), der an einer Oberfläche des vorderen Schichtstoffabschnitts (30) befestigt ist und parallel und anliegend am ersten Bein-Rand (34) vom Seiten-Rand (42) zu einem Rand des vorderen nichtelastischen Abschnitts (68) verläuft; und
einen zweiten Bein-Gummizug, der an einer Oberfläche des vorderen Schichtstoffabschnitts (30) befestigt ist und in zunehmendem Abstand vom ersten Bein-Gummizug (84) getrennt ist, da der Bein-Gummizug vom Seiten-Rand (42) zu einem Rand des vorderen nichtelastischen Abschnitts (68) verläuft.

13. Der absorbierende Artikel nach Anspruch 1, wobei die Durchgangsöffnungen innerhalb des Umfangs der Ultraschall-Bindungsstelle (116, 216) gebildet sind.

## Revendications

1. Article absorbant, comprenant :
une section stratifiée avant (30) définissant le bord d'extrémité avant (26), un bord d'entrejambe avant (27) parallèle et espacé longitudinalement du bord d'extrémité avant (26) et les premier et second bords latéraux opposés de manière transversale (42, 44) s'étendant dans des directions longitudinales, la section stratifiée avant (30) comprenant une partie non élastique avant (68) adjacente au bord d'entrejambe avant (27),
une partie élastique avant adjacente (24) au bord d'extrémité avant (26), les sections stratifiées avant (30) comprenant en outre :
un film polymère (62, 362),
une première couche non tissée (58, 358),
une section stratifiée arrière (32) définissant le bord d'extrémité arrière (66), un bord d'entrejambe arrière (29) parallèle et espacé longitudinalement à partir du bord d'extrémité arrière (66) et les premier et second bords latéraux opposés de manière transversale (50, 52) s'étendant dans des directions longitudinales, la section de stratifié arrière (32) comprenant une partie non élastique arrière (70) adjacente au bord d'entrejambe arrière (29) et une partie élastique arrière adjacente (24) au bord d'extrémité arrière (26) ; et
un ensemble absorbant (148) s'étendant longitudinalement entre le bord d'entrejambe avant (27) et le bord d'entrejambe arrière (29), l'ensemble absorbant (148) comprenant une feuille supérieure (154), une feuille inférieure (150) et un cœur absorbant (152) positionné entre la feuille supérieure (154) et la feuille inférieure (150) ;
**caractérisé en ce que** la section stratifiée avant (30) comprend en outre une pluralité de sites de liaison ultrasonore espacés (116, 216) fixant la première couche non tissée (58, 358) à un premier côté du film polymère (62, 362), dans lequel chaque site de liaison crée une région de bande (222) et forme une couche imperméable à l'intérieur du périmètre du site de liaison et une pluralité d'ouvertures de respiration (120, 320) à travers le film polymère (62, 362), dans lequel la pluralité d'ouvertures de respiration (120, 320) comprennent des déchirures (226) formant des passages traversants dans le stratifié avant qui permettent le passage de la vapeur d'eau (118) à travers le stratifié avant (30), chaque déchirure (226) étant formée dans le stratifié généralement à l'extérieur et adjacente à la région de bande (222) et
une seconde couche non tissée (104,304) fixée à un second côté du film polymère (62,362) opposée à la première couche non tissée (58, 358), dans lequel la seconde couche non tissée (104, 304) est fixée au film polymère (62,362) par un premier mécanisme d'adhésion dans une première zone (105), dans lequel le premier mécanisme d'adhésion est une pluralité de liaisons ultrasonores espacées (316), et un second mécanisme d'adhésion dans une seconde zone (107), dans lequel le second mécanisme d'adhésion est un matériau adhésif (363).

2. Article absorbant selon la revendication 1 dans lequel l'ensemble absorbant (148) chevauche (156, 158) la partie non élastique avant (68) et la partie non élastique arrière (70) et ne chevauche pas la partie élastique avant (24) et la partie élastique arrière (24).

3. Article absorbant selon la revendication 1, dans lequel la section stratifiée avant (30) définit en outre un premier bord de jambe (34), et l'article absorbant comprend en outre :
un premier élastique de jambe (84, 184) qui est collé à une surface de la section stratifiée avant (30) et s'étend parallèlement et de manière adjacente au bord de jambe (34) à partir du bord latéral (42) jusqu'à un bord de la partie non élastique avant (68) ; et
un second élastique de jambe qui est collé à une surface de la section stratifiée avant (30) et qui est de plus en plus séparé en distance du premier élastique de jambe (84, 184) étant donné que l'élastique de jambe s'étend à partir du bord latéral (42) jusqu'à un bord de la partie non élastique avant (68).

4. Article absorbant selon la revendication 3 dans lequel l'élastique de jambe (84,184) est positionné entre le film polymère (62, 362) et la seconde couche non tissée (104).

5. Article absorbant selon la revendication 1 dans lequel le film polymère (62 362) est un film élastomère.

6. Article absorbant selon la revendication 5 dans lequel la partie non élastique (68) du stratifié avant (30) est formée par la désactivation d'une partie du film élastomère.

7. Article absorbant selon la revendication 1 dans lequel les élastiques de jambe (84, 184) sont positionnés à l'intérieur de la seconde zone (107).

8. Article absorbant, comprenant :
une section stratifiée avant de respiration (30) définissant le bord d'extrémité avant (26), un bord d'entrejambe avant (27) parallèle et espacé longitudinalement du bord d'extrémité avant (26), les premier et second bords latéraux opposés de manière transversale (42, 44) s'étendant dans une direction longitudinale et les premier et second bords de jambe (34, 36), la section stratifiée avant (30) comprenant :
un film polymère (62, 362),
une première couche non tissée (58, 358) fixée à un premier côté du film polymère (62, 362) au niveau d'une pluralité de sites de liaison espacés (116, 216), dans lequel chaque site de liaison crée une région de bande (222) et forme une couche imperméable à l'intérieur du périmètre du site de liaison,
une seconde couche non tissée (104) fixée à un second côté du film polymère (62, 362) opposée à la première couche non tissée (58, 358),
une partie non élastique avant (68) adjacente au bord d'entrejambe avant (27) et une partie élastique avant (24) adjacente au bord d'extrémité avant (26), et
une pluralité d'ouvertures de respiration (120, 320) à travers le film polymère (68) ;
une section stratifiée arrière (32) définissant le bord d'extrémité arrière (28), un bord d'entrejambe arrière (29) parallèle et espacé longitudinalement du bord d'extrémité arrière (28) et les premier et second bords latéraux opposés de manière transversale (50, 52) s'étendant dans une direction longitudinale, et les premier et second bords de jambe (38, 40), la section stratifiée arrière (32) comprenant une partie non élastique arrière adjacente (70) au bord d'entrejambe arrière (29) et une partie élastique arrière (24) adjacente au bord d'extrémité arrière (28) ; et
un ensemble absorbant (148) s'étendant longitudinalement entre le bord d'entrejambe avant (27) et le bord d'entrejambe arrière (29), l'ensemble absorbant (148) comprenant une feuille supérieure (154), une feuille inférieure (150) et un cœur absorbant (152) positionné entre la feuille supérieure (154) et la feuille inférieure (150) ;
**caractérisé en ce que** la seconde couche non tissée (104) est fixée au film polymère (62, 362) par un premier mécanisme d'adhésion dans une première zone (105), dans lequel le premier mécanisme d'adhésion est une pluralité de liaisons ultrasonores espacées (316) et un second mécanisme d'adhésion dans une seconde zone (107), dans lequel le second mécanisme d'adhésion est un matériau adhésif (363), et
la pluralité d'ouvertures de respiration (120, 320) se compose d'une série de déchirures (226) formant des passages traversants qui permettent le passage de la vapeur d'eau (118) à travers le stratifié avant (30) chaque déchirure (226) étant formée dans le stratifié généralement à l'extérieur et adjacente à la région de bande (222).

9. Article absorbant selon la revendication 8, dans lequel les sites de liaison (116, 216) sont des sites de liaison ultrasonore.

10. Article absorbant selon la revendication 9, dans lequel les passages traversants sont formés à l'intérieur du périmètre du site de liaison ultrasonore (116, 216).

11. Article absorbant selon la revendication 9, dans lequel chaque déchirure (226) est formée à l'extérieur et adjacente à un périmètre du site de liaison ultrasonore (216).

12. Article absorbant selon la revendication 8, dans lequel la section stratifiée avant (30) définit en outre un premier bord de jambe (34), et l'article absorbant comprend en outre :
un premier élastique de jambe (84, 184) qui est collé à une surface de la section stratifiée avant (30) et s'étend parallèle et adjacent au bord de jambe (34) à partir du bord latéral (42) jusqu'à un bord de la partie non élastique avant (68) ; et
un second élastique de jambe qui est collé à une surface de la section stratifiée avant (30) et qui est de plus en plus séparé en distance du premier élastique de jambe (84) étant donné que les élastiques de jambe s'étendent à partir du bord latéral (42) jusqu'à un bord de la partie non élastique avant (68).

13. Article absorbant selon la revendication 1, dans lequel les passages traversants sont formés à l'intérieur du périmètre du site de liaison ultrasonore (116, 216).
